(19) Europäisches Patentamt / European Patent Office / Office européen des brevets

(11) **EP 2 764 869 A1**

(12) **EUROPEAN PATENT APPLICATION**
published in accordance with Art. 153(4) EPC

(43) Date of publication:
**13.08.2014 Bulletin 2014/33**

(51) Int Cl.:
***A61K 36/06*** *(2006.01)*     ***A61P 25/20*** *(2006.01)*
***A61K 31/7076*** *(2006.01)*

(21) Application number: **12838519.2**

(22) Date of filing: **09.10.2012**

(86) International application number:
**PCT/JP2012/076098**

(87) International publication number:
**WO 2013/051728 (11.04.2013 Gazette 2013/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2011 JP 2011222254**

(71) Applicant: **Lion Corporation**
**Tokyo 130-8644 (JP)**

(72) Inventors:
• **URADE, Yoshihiro**
**Kyoto-shi**
**Kyoto 606-0804 (JP)**

• **GOKITA, Toshio**
**Tokyo 130-8644 (JP)**
• **KAMADA, Ikuko**
**Tokyo 130-8644 (JP)**
• **SUZUKI, Kiwamu**
**Tokyo 130-8644 (JP)**
• **MURAKOSHI, Michiaki**
**Tokyo 130-8644 (JP)**

(74) Representative: **HOFFMANN EITLE**
**Patent- und Rechtsanwälte**
**Arabellastrasse 4**
**81925 München (DE)**

(54) **AGENT FOR IMPROVING QUALITY OF SLEEP**

(57) An object is to provide a sleep quality improving agent that has a sufficient effect of improving the quality of sleep, can exert the effect regardless of the form of administration, even by oral administration, and ensures safety for the body. A sleep quality improving agent containing an S-adenosylmethionine-containing yeast as an effective ingredient and a sleep quality improving composition containing the sleep quality improving agent are provided.

EP 2 764 869 A1

**Description**

**TECHNICAL FIELD**

The present invention relates to a sleep quality improving agent.

**BACKGROUND**

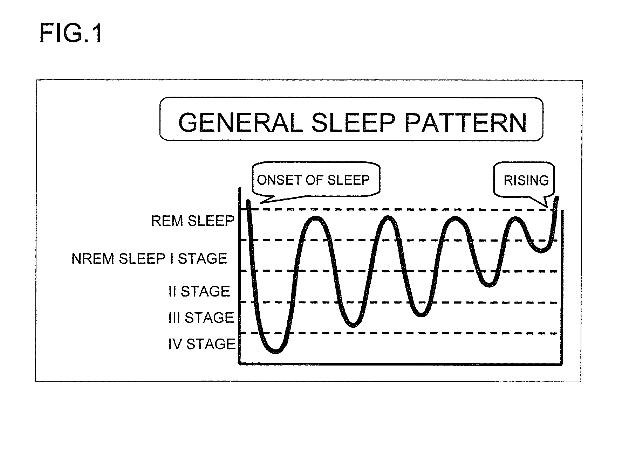

Sleep is divided into two broad types: NREM sleep (non-rapid eye movement sleep) in which the cerebrum almost stops its activities and REM sleep (rapid eye movement sleep) in which, although the whole body is in a relaxed state, part of the brain is highly active and dreaming occurs. These two sleep types are repeated at regular intervals, forming sleep (FIG. 1). To achieve good sleep quality, the time from the onset of sleep until appearance of NREM sleep (sleep onset latency) must be short, and sufficient NREM sleep time and deep NREM sleep, particularly deep NREM sleep at the initial stage of sleep, must be ensured.

However, in recent years, as 24-hour society becomes more prevalent, the average sleep time of the Japanese decreases significantly, and this causes an increase in the number of patients with sleep complaints. A survey on awareness of health build-up in Heisei 8 (1996) reports that 21.4% of people have some sleep complaints. The survey also reports that the NREM sleep time of elderly people is significantly shorter than that of young people and the quality of sleep deteriorates with aging.

Examples of the sleep complaints may include difficulty in falling asleep, nightmares, sleepiness on awakening in the morning, no feeling of sufficient sleep, residual fatigue even after awakening in the morning, and sleepiness during daytime. These may lead to a reduction in work efficiency and unexpected accidents. The causes of these sleep complaints include not only a short sleep time but also lack of good quality sleep. More specifically, the causes are that the time from the onset of sleep until appearance of NREM sleep (sleep onset latency) is long, the length of NREM sleep is short, and deep NREM sleep is not obtained. To address these sleep complaints, it is contemplated to use medical sleeping pills. However, to use sleeping pills, a diagnosis by a physician is required. Sleeping pills have side effects such as an unpleasant awakening, dysmnesia, and dependence. As described above, sleeping pills are often difficult to use easily and safely. Some pharmaceutical products, such as benzodiazepine-based sleeping pills and barbiruturic acid-based sleeping pills, reduce the amount of NREM sleep, and the quality of sleep can rather deteriorates unless these are used appropriately.

Besides the pharmaceutical products, sleep improving agents produced from natural ingredients and food and drink ingredients are actively researched and developed, and various sleep improving agents have been proposed. Examples of the effective ingredients of the proposed sleep improving agents may include components derived from plants of the Withania genus belonging to the Solanaceae family (Patent Literature 1), fermented products of Hemerocallis fulva. var. sempervirens (Patent Literature 2), theamine derived from tea leaves (Patent Literature 3), and ginseng extracts (Non Patent Literature 1). However, the effects of these ingredients on the sleep onset latency and the length and depth of NREM sleep are not clear, and their effects of improving the quality of sleep are not sufficient.

Other examples of the effective ingredients of the proposed sleep improving agents may include combinations of: B vitamins alone or E vitamins; a component selected from Bupleurum root, Cnidium officinale, Poria cocos, and extracts thereof; and S-adenosylmethionine (Patent Literature 4), and adenosine-containing compounds and adenosine-elevating compounds (Patent Literature 5). The effects of these ingredients on the sleep onset latency and the length and depth of NREM sleep are also not clear, and their effects of improving the quality of sleep are not sufficient.

It is known that the length of NREM sleep (stage 4) can be extended by intravenous injection or intramuscular injection of stabilized S-adenosylmethionine (i.e., S-adenosylmethionine disulfate tosylate) (Non Patent Literature 2). However, the S-adenosylmethionine disulfate tosylate has no influence on sleep onset latency and is not sufficient for improving the quality of sleep. In addition, only intravenous injection and intramuscular injection are shown as the administration method. Therefore, the S-adenosylmethionine disulfate tosylate is very inconvenient for practical use as a sleep quality improving agent because, for example, training is necessary for appropriate injection, a problem exists in terms of invasiveness because of pain during injection, and the formulation must be strictly managed in a microbial sense.

**RELATED ART DOCUMENTS**

**PATENT LITRATURE**

   Patent Literature 1: Japanese Patent Application Laid-Open No. 2006-28051

Patent Literature 2: Japanese Patent Application Laid-Open No. 2006-62998
Patent Literature 3: International Publication No. 2005/097101
Patent Literature 4: Japanese Patent Application Laid-Open No. 2007-277207
Patent Literature 5: Japanese Translation of PCT International Application No. 2006-513214

**NON-PATENT LITRATURE**

Non Patent Literature 1: Young Ho Rhee, et al., Psychopharmacology, 101, p.486-488 (1990)
Non Patent Literature 2: Monogr Gesamtgeb Psychiatr Psychiatry Ser, 18, 151-169, 1978

**SUMMARY OF THE INVENTION**

**PROBLEM TO BE SOLVED BY THE INVENTION**

The present invention has been made in view of the above circumstances, and it is an object to provide a sleep quality improving agent that has a sufficient effect of improving the quality of sleep, can exert the effect regardless of the form of administration, even by oral administration, and ensures safety for the body.

**MEANS FOR SOLVING PROBLEM**

The present inventors have conducted repeated studies to achieve the above object. Consequently, the inventors have found that an S-adenosylmethionine-containing yeast has the effect of improving the quality of sleep and thereby arrived at the present invention.

[1] A sleep quality improving agent comprising an S-adenosylmethionine-containing yeast as an effective ingredient.
[2] The sleep quality improving agent according to [1], wherein the sleep quality improving agent shortens sleep onset latency.
[3] The sleep quality improving agent according to [1] or [2], wherein the sleep quality improving agent deepens NREM sleep at an initial stage of sleep.
[4] The sleep quality improving agent according to any one of [1] to [3], wherein the sleep quality improving agent increases a percentage of NREM sleep time in a total sleep time.
[5] The sleep quality improving agent according to any one of [1] to [4], wherein the sleep quality improving agent improves less sleepiness feeling on awakening.
[6]. The sleep quality improving agent according to any one of [1] to [5], wherein the sleep quality improving agent improves ease of falling asleep and feeling of sound sleep.
[7] The sleep quality improving agent according to any one of [1] to [6], wherein the sleep quality improving agent improves dreaming.
[8] The sleep quality improving agent according to any one of [1] to [7], wherein the sleep quality improving agent improves feeling of fatigue.
[9] The sleep quality improving agent according to any one of [1] to [8], wherein the sleep quality improving agent is an oral agent.
[10] A sleep quality improving composition comprising the sleep quality improving agent according to any one of [1] to [9].
[11] The sleep quality improving composition according to [10], wherein the sleep quality improving composition is an oral agent.
[12] A method of use of an S-adenosylmethionine-containing yeast to improve quality of sleep.

**EFFECT OF THE INVENTION**

According to the present invention, a sleep quality improving agent that has a sufficient effect of improving the quality of sleep, can exert the effect regardless of the form of administration, even by oral administration, and ensures safety for the body is provided.

**BRIFE DESCRIPTION OF DRAWINGS**

FIG. 1 is a diagram illustrating a general sleep pattern.

FIG. 2 is a graph showing the cumulative amount of activity of mice 6 hours after sample administration in Example 1 and Comparative Examples 1 to 5.

FIG. 3 is a graph showing the percentage of each of sleep stages (wakefulness or NREM sleep) in Example 2 and Comparative Example 6.

FIG. 4 is a graph showing the percent change in sleep onset latency in Example 3 relative to that in Comparative Example 7.

FIG. 5 is a graph showing the percent change in the power value of delta waves at the initial stage of sleep in Example 3 relative to that in Comparative Example 7.

FIG. 6 is a graph showing the percent change in the score of factor I in Example 3 relative to that in Comparative Example 7.

FIG. 7 is a graph showing the percent change in the score of factor II in Example 3 relative to that in Comparative Example 7.

FIG. 8 is a graph showing the percent change in the score of factor III in Example 3 relative to that in Comparative Example 7.

FIG. 9 is a graph showing the percent change in the score of factor IV in Example 3 relative to that in Comparative Example 7.

FIG. 10 is a graph showing the percent change in the score of feeling of fatigue in Example 3 relative to that in Comparative Example 7.

## EMBODIMENTS FOR CARRYING OUT THE INVENTION

The sleep quality improving agent of the present invention comprises an S-adenosylmethionine-containing yeast as an effective ingredient. The S-adenosylmethionine-containing yeast is a yeast containing S-adenosylmethionine or its salt. Only one type of S-adenosylmethionine-containing yeast may be used, or a combination of two or more types may be used.

S-adenosylmethionine is a compound in which adenosine and methionine are coupled through a methylsulfonium bond. S-adenosylmethionine acts as a methyl group donor in a methylation reaction in a living body and plays an important role in promoting phospholipid metabolism and in methylating protein or nucleic acid. Examples of the salt of S-adenosylmethionine may include S-adenosylmethionine disulfate tosylate and S-adenosylmethionine chloride.

The S-adenosylmethionine-containing yeast may be a yeast originally containing S-adenosylmethionine or a yeast obtained by causing a yeast originally containing no S-adenosylmethionine to produce S-adenosylmethionine. Examples of the method of causing the yeast to produce S-adenosylmethionine may include a method in which the yeast is cultured to produce S-adenosylmethionine in the yeast (cells). In this method, the obtained culture solution may be used as the S-adenosylmethionine-containing yeast without any treatment, or the yeast collected after cultivation may be used as the S-adenosylmethionine-containing yeast. Alternatively, a dried S-adenosylmethionine-containing yeast obtained by drying the collected yeast after cultivation may be used. Preferably, in the present invention, the dried S-adenosylmethionine-containing yeast is used.

The S-adenosylmethionine-containing yeast may be a yeast that produces S-adenosylmethionine and can be administered orally. Examples of such a yeast may include yeasts of the Saccharomyces genus, and Saccharomyces Cerevisiae is preferred.

Various types of S-adenosylmethionine-containing yeasts are commercially available, and these commercial products may be used. Examples of the commercial products may include a "high-SAMe content dried yeast (trade name)" manufactured by MITSUBISHI GAS CHEMICAL COMPANY, INC. and "SupereSSe (trade name)" manufactured by Gnosis, Italy, and "AMee (trade name)" manufactured by IWATA CHEMICAL CO., LTD.

No particular limitation is imposed on the dosage of the sleep quality improving agent of the present invention so long as the effects of the invention are not impaired. The dosage may be appropriately changed according to factors such as the age and conditions of a living body to which the agent is administered. A preferred dosage of the sleep quality improving agent for obtaining the intended effect is generally 1 mg to 2,000 mg in terms of the amount of S-adenosylmethionine per day, preferably 10 mg to 1,600 mg, more preferably 10 mg to 1,000 mg, and still more preferably 20 mg to 800 mg. More specifically, when the contained amount of S-adenosylmethionine in the S-adenosylmethionine-containing yeast is, for example, 10% by mass, the dosage of the S-adenosylmethionine-containing yeast is generally 10 mg to 20,000 mg per day, preferably 100 mg to 16,000 mg, more preferably 100 mg to 10,000 mg, and still more preferably 200 mg to 8,000 mg. When the contained amount of S-adenosylmethionine in the S-adenosylmethionine-containing yeast is, for example, 5% by mass, the dosage of the S-adenosylmethionine-containing yeast is generally 20 mg to 40,000 mg per day, preferably 200 mg to 32,000 mg, more preferably 200 mg to 20,000 mg, and still more preferably 400 mg to 16,000 mg. The contained amount of S-adenosylmethionine or a salt thereof in the S-adenosylmethionine-containing yeast is 3% by mass or more, preferably 4% by mass or more, and still more preferably 5% by mass or more. No particular limitation is imposed on the upper limit, and a sufficient effect can be obtained even when the content is about 15% by weight or less.

The effective ingredient of the sleep quality improving agent of the present invention is the S-adenosylmethionine-containing yeast. The S-adenosylmethionine-containing yeast improves the quality of sleep more significantly than S-adenosylmethionine, and this will be actually proven in Example 1 and Comparative Example 5 described later. (The reason that a salt of S-adenosylmethionine was used in Comparative Example 5 is that S-adenosylmethionine alone is unstable.) As will be shown in Comparative Example 4, a combination of a yeast containing no S-adenosylmethionine and a salt of S-adenosylmethionine does not have the effect of improving the quality of sleep. The reason that the S-adenosylmethionine-containing yeast has the effect of improving the quality of sleep is unclear. For example, the reason may be that the form of absorption from the digestive tract in the body of the yeast becomes favorable.

The sleep quality improving agent of the present invention in its original form may be formulated into a preparation and used as a final product such as a food or drink product, a pharmaceutical product, or a quasi-pharmaceutical product. In addition, the sleep quality improving agent can be used as additives for food and drink products, additives for pharmaceutical products, and additives for quasi-pharmaceutical products. In this manner, the effect of improving the quality of sleep can be imparted to food and drink products, pharmaceutical products, and quasi- pharmaceutical products.

It is sufficient that the sleep quality improving agent of the present invention comprises the S-adenosylmethionine-containing yeast as the effective ingredient. The sleep quality improving agent may contain additional components other than the S-adenosylmethionine-containing yeast. Examples of the additional components may include components for ensuring stability mainly during storage and distribution such as a storage stabilizer. One or two or more types (preferably approximately one to three types and more preferably approximately one type) of components selected from the components constituting a target final product such as a food or drink product, a pharmaceutical product, or a quasi-pharmaceutical product may be added in advance to the sleep quality improving agent.

The sleep quality improving agent of the present invention may be combined with a component other than the S-adenosylmethionine-containing yeast to prepare a sleep quality improving composition.

No particular limitation is imposed on the component, other than the S-adenosylmethionine-containing yeast, contained in the sleep quality improving composition of the present invention, so long as the object of the invention is not impaired. Examples of such a component may include pharmaceutically acceptable additives such as excipients, disintegrators, binding agents, lubricants, coating agents, coloring agents, color formers, taste masking agents, flavoring agents, antioxidants, antiseptics, taste agents, acidulants, sweeteners, fortifiers, vitamin compounds, inflating agents, thickeners, and surfactants. One or two or more types of additives that do not impair the effect of improving the quality of sleep and the characteristics necessary for the formulation such as the stability of the formulation and are appropriate for the dosage form of the final product may be selected from the above additives. The components other than the S-adenosylmethionine-containing yeast may be components having the effect of improving the quality of sleep other than that of the S-adenosylmethionine-containing yeast. The sleep quality improving composition of the present invention may comprise one type of component other than the S-adenosylmethionine-containing yeast or a combination of two or more types of such components.

The dosage of the sleep quality improving composition of the present invention may be controlled such that the amount of S-adenosylmethionine is within the range described above for the dosage of the sleep quality improving agent of the present invention.

The sleep quality improving composition of the present invention in its original form may be used as a final product such as a food or drink product, a pharmaceutical product, or a quasi-pharmaceutical product. The composition may also be used as additives for food and drink products, additives for pharmaceutical products, and additives for quasi-pharmaceutical products. In this manner, the effect of improving the quality of sleep can be imparted to food and drink products, pharmaceutical products, and quasi-pharmaceutical products.

No particular limitation is imposed on the form of administration of the sleep quality improving agent of the present invention and the sleep quality improving composition of the present invention. Examples of the form of administration may include oral administration such as buccal administration and sublingual administration and parenteral administration such as intravenous administration, intramuscular administration, subcutaneous administration, percutaneous administration, transnasal administration, and pulmonary administration. Of these, less invasive administration forms are preferred, and oral administration is more preferred.

No particular limitation is imposed on the dosage forms of the sleep quality improving agent of the present invention and the sleep quality improving composition of the present invention. Examples of the dosage form for oral administration may include a liquid form (liquid agents), a syrup form (syrup agents), a solid form (tablets), a capsule form (capsules), a powdery form (granules and fine particles), a soft capsule form (soft capsules), a semi-liquid form, a cream form, and a paste form. Examples of the dosage form for parenteral administration may include liquid agents such as injections and nasal drops and an atomized form such as sprays and inhalants.

No particular limitation is imposed on the time of administration of the sleep quality improving agent of the present invention and the sleep quality improving composition of the present invention. These are administered generally before going to bed, preferably between three hours before going to bed and the time of going to bed, more preferably between two hours before going to bed and the time of going to bed, still more preferably between one hour before going to bed and the time of going to bed, and particularly preferably one hour before going to bed.

The S-adenosylmethionine-containing yeast, which is the effective ingredient of the sleep quality improving agent of the present invention, has a significant effect of improving the quality of sleep.

The "quality of sleep" in the present invention means that sleep gives the tired body and brain a rest. Even when the sleep time is long, fatigue cannot be sufficiently relieved if the quality of sleep is low. Examples of the index for measuring the quality of sleep may include the time from the onset of sleep until appearance of NREM sleep (hereinafter referred to as sleep onset latency), the depth of NREM sleep at the initial stage of sleep, the percentage of NREM sleep time in the total sleep time, less sleepiness feeling on awakening, ease of falling asleep and feeling of sound sleep, dreaming, satisfaction with the length of sleep, and feeling of fatigue. Of these, the sleep onset latency, the depth of NREM sleep at the initial stage of sleep, the percentage of NREM sleep time in the total sleep time, less sleepiness feeling on awakening, ease of falling asleep and feeling of sound sleep, dreaming, and the feeling of fatigue are preferred. The NREM sleep at the initial stage of sleep means NREM sleep that appears immediately after the onset of sleep and before appearance of REM sleep. The sleep onset latency can be checked using an record of brain waves obtained by measuring brain waves during sleep, as will be shown in Examples. The depth of NREM sleep at the initial stage of sleep can be checked using the power value of delta waves in the brain waves during sleep, as will be shown in Examples. As the power value of delta waves increases, the depth of sleep increases. The percentage of NREM sleep time in the total sleep time can also be checked using the electroencephalogram record obtained by measuring brain waves during sleep, as will be shown in Examples. Less sleepiness feeling on awakening, ease of falling asleep and the feeling of sound sleep, dreaming, the satisfaction of the length of sleep, and the feeling of fatigue can be evaluated using an OSA sleep inventory MA version (YAMAMOTO Yukari, TANAKA Hideki, TAKASE Miki, YAMAZAKI Katsuo, AZUMI Kazuo, and SHIRAKAWA Shuichiro: Standardization of revised version of OSA sleep inventory for middle age and aged, Brain science and mental disorders, 10: 401-409, 1999), as will be shown in Examples. The feeling of fatigue can be evaluated using a Visual Analogue Scale (VAS) test sheet recommended by Japanese Society of Fatigue Science and shown in the guidelines on the method of evaluating the feeling of fatigue in anti-fatigue clinical evaluation of foods for specified health uses by the Japanese Society of Fatigue Science, as will be shown in Examples.

In the present invention, the phrase "to improve the quality of sleep" means that the quality of sleep is improved or raised. The improvement in the quality of sleep can be checked using, for example, at least one selected from the group consisting of a reduction in sleep onset latency, deep NREM sleep at the initial stage of sleep, an increase in the percentage of NREM sleep time in the total sleep time, an improvement in terms of less sleepiness feeling on awakening, an improvement in ease of falling asleep and feeling of sound sleep, an improvement in dreaming (for example, no frequent dreaming and no nightmare), and an increase in the feeling of recovery from fatigue (mitigation of the feeling of fatigue). The improvement in the quality of sleep can be checked using the conditions shown in Examples.

According to the sleep quality improving agent of the present invention, the quality of sleep can be effectively improved. S-adenosylmethionine is a material naturally contained in a living body. Therefore, the sleep quality improving agent of the present invention containing S-adenosylmethionine as the effective ingredient is safe for the living body, and the user can use the sleep quality improving agent with a sense of security. In addition, the sleep quality improving agent of the present invention can reduce the time of nocturnal awakenings during sleep to maintain the time of sleep as will be shown in the Examples below, so that the effect on recovery from fatigue is more significantly exerted. Therefore, the sleep quality improving agent is expected to be applied to prevention and therapy of diseases related to a reduction in the quality of sleep such as diabetes, heart disease, hypertension, hyperlipidemia, and Alzheimer's disease. Accordingly, the sleep quality improving agent of the present invention is useful as final products such as food and drink products, pharmaceutical products, and quasi-pharmaceutical products.

No particular limitation is imposed on the subjects who ingest the sleep quality improving agent of the present invention or the sleep quality improving composition of the present invention. Examples of the subjects may include subjects who sleep poorly, subjects who are still sleepy on awakening, subjects who have a difficulty in falling asleep, subjects who cannot sleep soundly (who feel that they cannot sleep deeply), subjects who have a bad dream, subjects who feel fatigue, and subjects who feel fatigue even after sleep. In addition, subjects who do not have any particular problem can ingest the sleep quality improving agent on a daily basis for the purpose of improving or maintaining the quality of sleep.

Preferably, the sleep quality improving agent of the present invention is used as various food and drink products. Examples of the food and drink products may include beverages such as soft drinks, carbonated drinks, energy drinks, powdered drinks, fruit drinks, milk-based drinks, and jelly drinks, confectioneries such as cookies, cakes, chewing gums, candies, tablets, gummies, "manjyu (steamed buns with filing)," "yokan (adzuki bean jellies)," puddings, jellies, ice creams, and sherbets, processed marine products such as "kamaboko (boiled fish pastes)," "chikuwa (baked tubular rolls of fish pastes)," and "hanpenn (boiled cakes made of ground fish),", processed livestock products such as hamburgers, hams, sausages, wiener sausages, cheeses, butters, yogurt, fresh creams, margarines, and fermented milk, soups such as powdered soups and liquid soups, staple foods such as rice, noodles (dried noodles, fresh noodles), breads, and cereals, and seasonings such as mayonnaise, shortening, dressing, sauce, "tare (dipping sauce)," and soy sauce. In addition, the sleep quality improving agent of the present invention may be used as food and drink products such as health foods, functional foods, dietary supplements (supplements), foods for specified health uses, foods for medical use, foods for the sick, infant foods, foods for nursing care, and foods for the elderly. Of these, the sleep quality improving agent is preferably used as health foods and functional foods.

**EXAMPLES**

The present invention will next be described by way of Examples.

1. Evaluation of sleep inducing effect ((Example 1 and Comparative Examples 1 to 5: Measurement of amount of activity of mice)

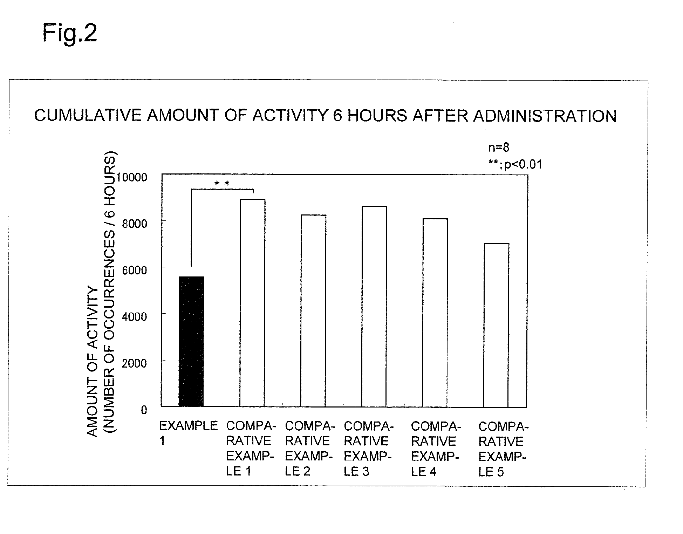

To check the sleep inducing effect of the S-adenosylmethionine-containing yeast, the following test was performed using respective samples shown in TABLE 1.
The S-adenosylmethionine-containing yeast (the sample in Example 1) used was "AMee (trade name)" manufactured by IWATA CHEMICAL CO., LTD. "Nissin Super Camellia dry yeast (trade name)" manufactured by Nisshin Foods Inc. was used as a yeast containing no adenosylmethionine (the sample in Comparative Example 2). S-adenosylmethionine disulfate tosylate (the sample in Comparative Example 3) used was S-adenosylmethionine disulfate tosylate manufactured by CHEMaster. The sample in Comparative Example 2 and the sample in Comparative Example 3 were mixed and used as a mixture of the yeast containing no S-adenosylmethionine and S-adenosylmethionine disulfate tosylate (the sample in Comparative Example 4). S-adenosylmethionine chloride used in Comparative Example 5 (the sample in Comparative Example 5) was a reagent from SIGMA. In each of Example 1 and Comparative Examples 2, 3, 4, and 5, a suspension of the sample in a 0.5% by mass aqueous methyl cellulose solution was prepared and used as a sample solution for an experiment. A 0.5% by mass aqueous methyl cellulose solution (the sample in Comparative Example 1) was used as a control solution.
When a sample has the function of inducing sleep, the amount of activity always decreases after administration of the sample. Therefore, the sleep inducing effect of each sample was evaluated by determining whether or not the administration of the sample solution to a mouse caused a reduction in the amount of activity.
8-Week old or 9-week old male C57BL/6 mice were purchased from Japan SLC, Inc. and acclimatized for 3 to 6 days. The amount of activity during acclimatization was used to divide the mice into administration groups. Immediately before the onset of the dark period, a sample solution or the control solution was orally administered in an amount of 10 mL/kg per body weight, and the amount of activity was measured for 24 hours after administration. Each administration group included 8 mice (n = 8).
The amount of activity was measured under the following conditions. The mice were raised in individual cages, and an infrared camera was disposed above each cage. The imaging range of the infrared camera was divided into 64 sections, and the number of occurrences that the mouse crossed a section was measured. The number of occurrences was collected every 30 minutes.
TABLE 1 shows the type of sample, the amount of oral administration of the sample solution, and the cumulative amount of activity (the number of occurrences / 6 hours) 6 hours after administration for each of Example 1 and the Comparative Examples. FIG. 2 shows the cumulative amount of activity of mice 6 hours after sample administration for each of the Example and Comparative Examples. In FIG. 2, "**" represents that a significant difference was found at $p < 0.01$.
[TABLE 1]

TABLE 1 (TYPE OF SAMPLE, AMOUNT OF ORAL ADMINISTRATION OF SAMPLE SOLUTION, AND CUMULATIVE AMOUNT OF ACTIVITY 6 HOURS AFTER ADMINISTRATION)

| | EXAMPLE 1 | COMPARATIVE EXAMPLE 1 | COMPARATIVE EXAMPLE 2 | COMPARATIVE EXAMPLE 3 | COMPARATIVE EXAMPLE 4 | COMPARATIVE EXAMPLE 5 |
|---|---|---|---|---|---|---|
| | S-ADENOSYLMETHIONINE-CONTAINING YEAST | CONTROL SOLUTION (0.5% BY MASS AQUEOUS METHYL CELLULOSE SOLUTION) | YEAST CONTAINING NO S-ADENOSYLMETHIONINE | S-ADENOSYL-METHIONINE DISULFATE TOSYLATE | YEAST CONTAINING NO S-ADENOSYLMETHIONINE + S-ADENOSYLMETHIONINE DISULFATE TOSYLATE | S-ADENOSYLMETHIONINE CHLORIDE |
| AMOUNT OF ORAL ADMINISTRATION: mg/kg (IN TERMS OF S-ADENOSYLMETHIONINE: mg/kg) | 3000 (150) | - (0) | 3000 (0) | 293 (150) | 3000 +293 (150) | 150 (137) |
| CUMULATIVE AMOUNT OF ACTIVITY 6 HOURS AFTER ADMINISTRATION (NUMBER OF OCCURRENCES / 6 HOURS) | 5583 | 8917 | 8250 | 8618 | 8019 | 7068 |

The following can be seen from TABLE 1 and FIG. 2.

In Example 1 in which the S-adenosylmethionine-containing yeast was used as the sample, the cumulative amount of activity 6 hours after administration was significantly lower than that in Comparative Example 1 in which the control solution was administered. However, no significant difference was found between the cumulative amount of activity 6 hours after administration in Comparative Example 1 and the cumulative amount of activity 6 hours after administration in each of Comparative Examples 2 to 5. Particularly, in Comparative Example 2 in which the yeast containing no S-adenosylmethionine was administered, the cumulative amount of activity 6 hours after administration was almost the same as that in Comparative Example 1 in which the control solution was administered.

These results show that although the yeast containing no S-adenosylmethionine has no sleep inducing effect, the S-adenosylmethionine-containing yeast has a sleep inducing effect. These results also show that S-adenosylmethionine disulfate tosylate and S-adenosylmethionine chloride have no sleep inducing effect.

2. Evaluation 1 of the effect of improving the quality of sleep (Example 2 and Comparative Example 6: Measurement of brain waves of mice)

To check the effect of the S-adenosylmethionine-containing yeast on the quality of sleep, the following experiment was performed to measure brain waves. More specifically, electrodes were attached to the head of a mouse, and the brain waves of the mouse that was allowed to move freely in a recording chamber were recorded to measure the time of "wake," the time of "REM sleep," and the time of "NREM sleep."

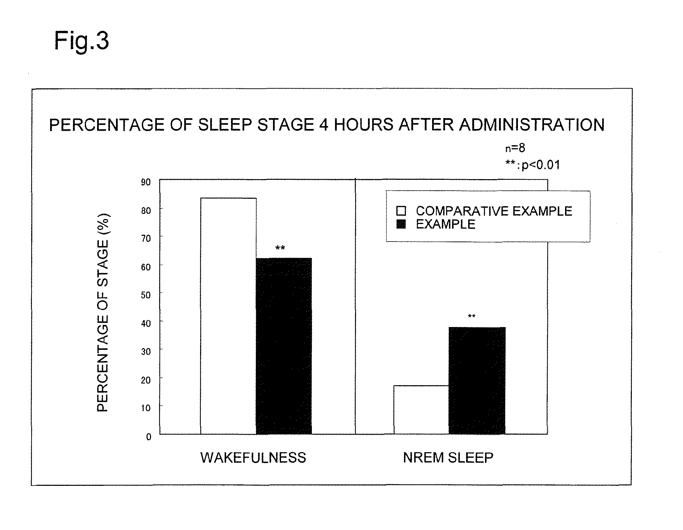

Electrodes for the brain waves and an electromyogram were attached to each of 8-week old male C57BL/6 mice purchased from Japan SLC, Inc. After the electrodes were attached, each mouse was recovered in a recovering chamber for 10 days. Then the mouse was transferred to the recording chamber, and cables were attached to the mouse. Brain wave analysis software SleepSign (registered trademark) Ver 3.0 (KISSEI COMTEC Co., Ltd.) was used to check whether or not the brain waves could be recognized, and the mouse was acclimatized for 3 days. Then the brain waves were measured for 24 hours to check whether or not a sleep-wakefulness rhythm was maintained, and the mice were divided into administration groups. Each administration group included 8 mice (n = 8). Immediately before the onset of the dark period, a suspension prepared by suspending the S-adenosylmethionine-containing yeast in a 0.5% by mass aqueous methyl cellulose solution was orally administered in an amount of 3 g/kg in terms of the S-adenosylmethionine-containing yeast, or a control solution was orally administered in an amount of 10 mL/kg. Then the brain waves were recorded for 24 hours. The control solution was a 0.5% by mass aqueous methyl cellulose solution. After recording, the brain waves were automatically analyzed by the SleepSign (registered trademark) Ver 3.0, and the evaluator checked the results of the automatic analysis to divide the sleep into three sleep stages: a wakefulness stage, a REM sleep stage, and a NREM sleep stage. The ratio of the time of each sleep stage to the total brain wave measurement time (4 hours) was computed as a percentage and used as the percentage of the sleep stage 4 hours after administration. The average for 8 mice was computed (TABLE 2 and FIG. 3).

[TABLE 2]

TABLE 2 (RESULTS OF BRAIN WAVE MEASUREMENT (AVERAGE FOR 8 MICE))

| RATIO OF SLEEP STAGE 4 HOURS AFTER ADMINISTRATION (%) | EXAMPLE 2 | COMPARATIVE EXAMPLE 6 |
|---|---|---|
| | S-ADENOSYLMETHIONINE-CONTAINING YEAST | CONTROL SOLUTION (0.5% BY MASS AQUEOUS METHYL CELLULOSE SOLUTION) |
| WAKEFULLNESS | 62 (%) | 83 (%) |
| NREM SLEEP | 38 (%) | 17 (%) |

The following can be seen from TABLE 2 and FIG. 3. In Example 2 in which the S-adenosylmethionine-containing yeast was used as the sample, the wakefulness time was significantly reduced as compared with that in Comparative Example 6 in which the control solution was administered, and the NREM sleep was significantly increased. The percentage of REM sleep was 1% in Example 2 and 0.7% in Comparative Example 6, and no difference was found.

These results show that the quality of sleep is improved because the administration of the S-adenosylmethionine-containing yeast causes an increase in the percentage of NREM sleep time in the total sleep time. The results also show that nocturnal awakenings can be reduced and the sleep time can be maintained.

3. Evaluation 2 of the effect of improving the quality of sleep (Example 3 and Comparative Example 7: Measurement of human brain waves and survey of feelings about sleep)

To check the effect of the S-adenosylmethionine-containing yeast on the quality of sleep of human, the following test was performed using samples shown in TABLE 3.

Tablets containing the S-adenosylmethionine-containing yeast (the content of S-adenosylmethionine in 9 tablets: 88 mg) were prepared as the sample in Example 3. Placebo tablets containing no S-adenosylmethionine-containing yeast were prepared as the sample in Comparative Example 7. The details of these compositions are as shown in TABLE 3.

[TABLE 3]

TABLE 3 (COMPOSITION OF TABLETS)

| COMPONENT (AMOUNT PER SINGLE DOSE: AMOUNT OF COMPONENT IN 9 TABLETS, mg) | SAMPLE IN EXAMPLE 3 | SAMPLE IN COMPARATIVE EXAMPLE 7 |
|---|---|---|
| S-ADENOSYLMETHIONINE-CONTAINING YEAST (CONTENT IN TERMS OF S-ADENOSYLMETHIONINE) | 1530 (88) | 0 |
| EXCIPIENT (CRYSTALLINE CELLULOSE) | 270 | 1800 |
| TOTAL AMOUNT | 1800 | 1800 |

12 Adult male and female panelists with relatively bad sleep conditions were selected according to a survey performed in advance using the Pittsburgh Sleep Quality Questionnaire. Each of the 12 panelists was requested to orally ingest the sample in the above Example 3 one hour before going to bed, and a two-electrode type portable brain wave measurement device was attached to the panelist to measure the brain waves during sleep. Similarly, each of the same 12 panelists was requested to orally ingest the sample in Comparative Example 7 one hour before going to bed, and the two-electrode type portable brain wave measurement device was attached to the panelist to measure the brain waves during sleep. In the next morning, the panelists answered a questionnaire on feelings about sleep and a VAS test sheet for the feeling of fatigue. The number of times of administration of each sample and the number of measurements performed were four times for each panelist. The average sleep time of the panelists was 6.5 hours. The difference in sleep time between the panelist with the shortest sleep time and the panelist with the longest sleep time was 2 hours, and the difference was not significant.

(Brain wave analysis)

The analysis of the brain waves was entrusted to SleepWell Co., Ltd. The time from the onset of sleep (the start of brain wave measurement) until appearance of NREM sleep was used as sleep onset latency. The depth of sleep can be determined by the power value of delta waves in the brain waves during sleep. The larger the power value of delta waves is, the larger the depth of sleep is. Immediately after the onset of sleep, NREM sleep appears, and then REM sleep appears. The power value of delta waves during NREM sleep immediately after the onset of sleep until appearance of REM sleep was used as the power value of delta waves at the initial stage of sleep.

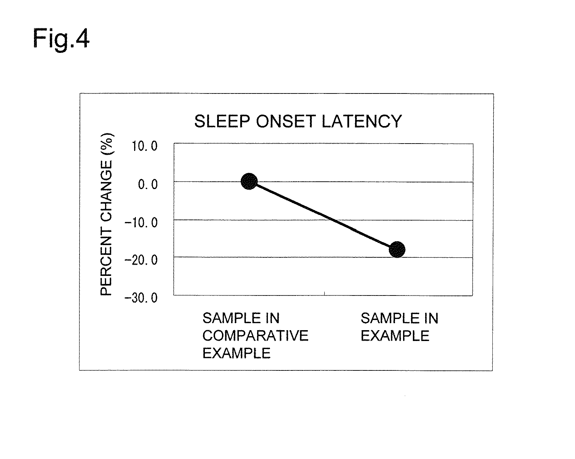

The values of sleep onset latency vary depending on individual panelists. Therefore, the sleep onset latency was measured four times for each panelist, and the average of the four measurements was computed. Then the percent change (%) in sleep onset latency relative to that when the placebo was ingested was computed for each panelist using the following formula (1) (TABLE 4 and FIG. 4).

[Formula (1)]

Percent change (%) in sleep onset latency =

{(average sleep onset latency value after ingestion of S-adenosylmethionine-containing yeast) / (average sleep onset latency value after ingestion of placebo)} × 100 - 100

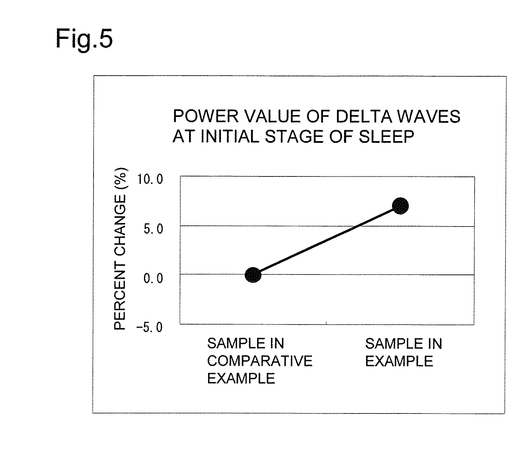

The power values of delta waves also vary depending on individual panelists. Therefore, the power value of delta waves was measured four times for each panelist, and the average of the four measurements was computed. Then the percent change (%) in the power value of delta waves relative to that when the placebo was ingested was computed for each panelist using the following formula (2) (TABLE 4 and FIG. 5).

[Formula (2)]

Percent change (%) in power value of delta waves =

{(average power value of delta waves after ingestion of S-adenosylmethionine-containing yeast) / (average power value of delta waves after ingestion of placebo)} × 100 - 100

(Survey of feelings about sleep)

OSA sleep inventory MA version (YAMAMOTO Yukari, TANAKA Hideki, TAKASE Miki, YAMAZAKI Katsuo, AZUMI Kazuo, and SHIRAKAWA Shuichiro: Standardization of revised version of OSA sleep inventory for middle age and aged, Brain science and mental disorders, 10: 401-409, 1999) was used for a survey of feelings about sleep. The OSA sleep inventory MA version includes 20 questions and is an evaluation method for evaluating factor I: sleepiness feeling on awakening (i.e., less sleepiness feeling on awakening), factor II: initiation and maintenance of sleep (i.e., ease of falling asleep and an improvement in soundness of sleep), factor III: dreaming (i.e., having a good dream), and factor IV: recovery from fatigue (i.e., lack of the feeling of fatigue).

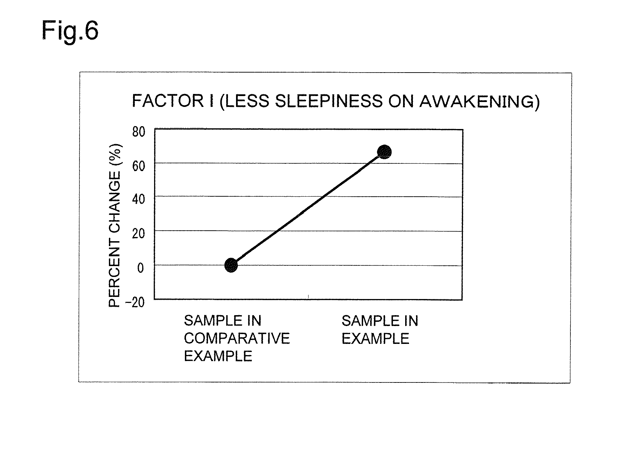

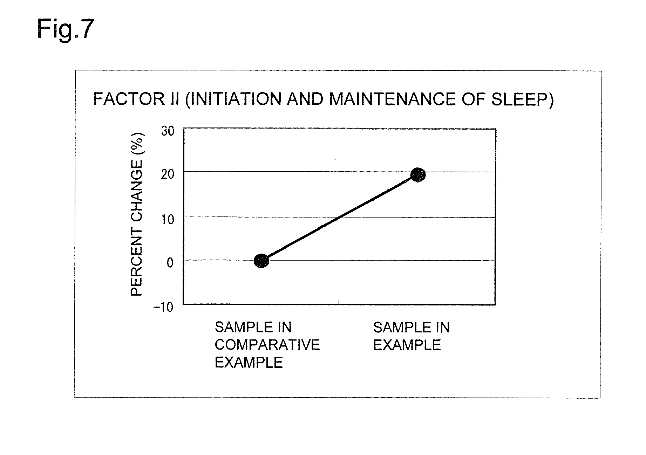

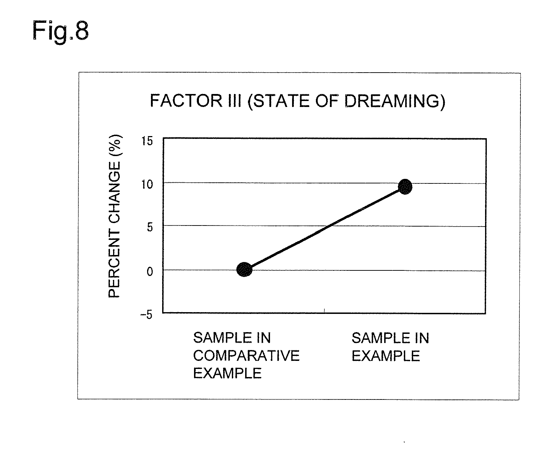

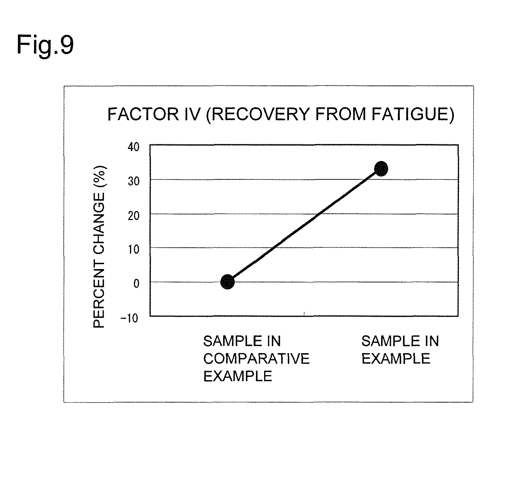

The scores of the respective factors were computed, and the average values of four measurements were computed for each panelist for each ingested sample. The percent change (%) in the score of each factor relative to that when the placebo was ingested was computed for each panelist using the following formula (3). The average percent change for the 12 panelists was computed (TABLE 5 and FIGs. 6 to 9).

[Formula (3)]

Percent change (%) in score of each factor =

{(average value after ingestion of S-adenosylmethionine-containing yeast) / (average value after ingestion of placebo)} × 100 – 100

(Survey on feeling of fatigue)

A Visual Analogue Scale (VAS) test sheet recommended by Japanese Society of Fatigue Science and shown in the guidelines on the method of evaluating the feeling of fatigue in anti-fatigue clinical evaluation of foods for specified heath uses by the Japanese Society of Fatigue Science was used for the survey on the feeling of fatigue. More specifically, the left end of a horizontal straight line with a certain length was set to "the best feeling with no fatigue," and the right end was set to "the worst feeling with exhaustion without motivation to do anything." Each panelist marked a cross on the straight line as the answer to the feeling of fatigue on awakening in the morning with reference to the feeling shown at the left and right ends of the straight line.

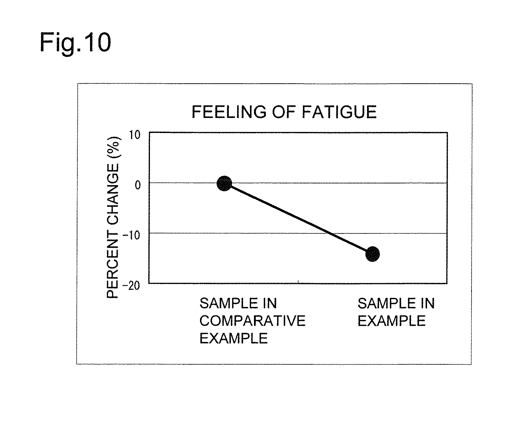

The distance from the left end of the straight line to the cross was measured to judge the feeling of fatigue. Specifically, it was judged that the smaller the value of the distance is, the less the feeling of fatigue is. The measurement was performed four times for each panelist for each ingested sample, and the average of the measurements was computed. The percent change (%) relative to placebo ingestion was computed for each panelist using the following formula (4). Then the average percent change for the 12 panelists was computed (TABLE 6 and FIG. 10).

[Formula (4)]

Percent change (%) in feeling of fatigue = {(average value after ingestion of S-adenosylmethionine-containing yeast) / (average value after ingestion of placebo)} × 100 − 100

(Results of brain wave analysis)

[TABLE 4]

TABLE 4 RESULTS FOR SLEEP ONSET LATENCY AND NREM SLEEP AT INITIAL STAGE OF SLEEP (AVERAGE OF PERCENT CHANGES FOR 12 PANELISTS)

|  | SAMPLE IN EXAMPLE 3 | SAMPLE IN COMPARATIVE EXAMPLE 7 |
|---|---|---|
| SLEEP ONSET LATENCY | -18.1% | 0% |
| POWER VALUE OF DELTA WAVES AT INITIAL STAGE OF SLEEP | 7.1% | 0% |

The following can be seen from TABLE 4 and FIGs. 4 and 5. In Example 3 in which the tablets of the S-adenosylmethionine-containing yeast were orally ingested as the sample, the sleep onset latency was shorter than that in Comparative Example 7 in which the placebo tablets were orally ingested. In addition, the power value of delta waves at the initial stage of sleep was large, and NREM sleep at the initial stage of sleep was sufficiently deep.

These results show that administration of the S-adenosylmethionine-containing yeast shortens sleep onset latency, deepens NREM sleep at the initial stage of sleep, and improves the quality of sleep effectively.

(Results of survey of feelings about sleep)

[TABLE 5]

TABLE 5 RESULTS OF SURVEY OF FEELINGS ABOUT SLEEP USING OSA SLEEP INVENTORY MA VERSION (PERCENT CHANGE IN EACH FACTOR (AVERAGE FOR 12 PANELISTS))

|  | SAMPLE IN EXAMPLE 3 | SAMPLE IN COMPARATIVE EXAMPLE 7 |
|---|---|---|
| FACTOR I (LESS SLEEPINESS FEELING ON AWAKENING) | 66.6% | 0% |
| FACTOR II (INITIATION AND MAINTENANCE OF SLEEP) | 19.4% | 0% |
| FACTOR III (STATE OF DREAMING) | 9.5% | 0% |
| FACTOR IV (RECOVERY FROM FATIGUE) | 33.0% | 0% |

The following can be seen from TABLE 5 and FIGs. 6 to 9. In Example 3 in which the tablets of the S-adenosylmethionine-containing yeast were orally ingested as the sample, the percent change in factor I (less sleepiness feeling on awakening) was higher than that in Comparative Example 7 in which the placebo tablets were orally ingested. The percent change in the score of factor II (initiation and maintenance of sleep), the percent change in factor III (state of dreaming), and the percent change in factor IV (recovery from fatigue) were similar to the percent change in factor I. These results show that the effect of improving the quality of sleep was exerted by the administration of the S-adenosylmethionine-containing yeast to thereby improve sleepiness on awakening. These results also show that the effect of improving the quality of sleep was exerted to thereby improve ease of falling asleep and feeling of sound sleep. These results also show that the effect of improving the quality of sleep was exerted to thereby improve the state of dreaming. In addition, these results show that the effect of improving the quality of sleep was exerted to thereby increase the feeling of recovery from fatigue.

(Results of survey on feeling of fatigue)

[TABLE 6]

TABLE 6 RESULTS OF VAS TEST (PERCENT CHANGE IN FEELING OF FATIGUE (AVERAGE FOR 12 PANELISTS))

|  | SAMPLE IN EXAMPLE 3 | SAMPLE IN COMPARATIVE EXAMPLE 7 |
|---|---|---|
| FEELING OF FATIGUE | -14.1% | 0% |

The following can be seen from TABLE 6 and FIG. 10. In Example 3 in which the tablets of the S-adenosylmethionine-containing yeast were orally ingested as the sample, a change to minus in comparison with Comparative Example 7 in which the placebo tablets were orally ingested was found. These results show that the effect of improving the quality of sleep was exerted by the administration of the S-adenosylmethionine-containing yeast, so that the feeling of fatigue disappeared (the feeling of fatigue was mitigated).

Prescription Examples of the sleep quality improving agent of the present invention and the sleep quality improving composition of the present invention are shown below.

(Prescription Example 1: Tablet (1))

290 mg of the S-adenosylmethionine-containing yeast and 5 mg of calcium stearate were subjected to tablet compression in a conventional manner and produced tablets.

(Prescription Example 2: Tablet (2))

250 mg of the S-adenosylmethionine-containing yeast, 1.6 mg of pyridoxine hydrochloride, 10 $\mu$g of cyanocobalamin, 33 $\mu$g of folic acid, 45 mg of crystalline cellulose, and 5 mg of calcium stearate were subjected to tablet compression in a conventional manner and produced tablets.

(Prescription Example 3: Tablet (3))

250 mg of the S-adenosylmethionine-containing yeast, 100 $\mu$g of pyridoxine hydrochloride, 0.2 $\mu$g of cyanocobalamin, 12 $\mu$g of folic acid, 45 mg of crystalline cellulose, and 5 mg of calcium stearate were subjected to tablet compression in a conventional manner and produced tablets.

(Prescription Example 4: Tablet (4))

Tablets were produced in a conventional manner using the following raw materials.

A granulated product (108 mg), the S-adenosylmethionine-containing yeast (20 mg), sorbitol (110 mg), partially pregelatinized modified starch (15 mg), magnesium phosphate (75 mg), calcium stearate (3 mg), menthol particles (40 mg), and aspartame (5 mg) were subjected to tablet compression in a conventional manner and produced tablets.

The above granulated product was produced by adding a 6% by mass aqueous hydroxypropyl cellulose solution (4,000 g) to erythritol (1,935 g) and cornstarch (300 g). The average particle diameter of the granulated product was 290 $\mu$m.

(Prescription Example 5: Soft capsules)

Soft capsules were produced in a conventional manner using the following raw materials.

First, the following raw materials were mixed to prepare a filling: the S-adenosylmethionine-containing yeast (20 mg), vegetable oil (110 mg), glycerine fatty acid ester (10 mg), and beeswax (10 mg).

Soft capsules were produced using the filling and pork gelatin.

**Claims**

1. A sleep quality improving agent comprising an S-adenosylmethionine-containing yeast as an effective ingredient.

2. The sleep quality improving agent according to claim 1, wherein the sleep quality improving agent shortens sleep onset latency.

3. The sleep quality improving agent according to claim 1 or 2, wherein the sleep quality improving agent deepens NREM sleep at an initial stage of sleep.

4. The sleep quality improving agent according to any one of claims 1 to 3, wherein the sleep quality improving agent increases a percentage of NREM sleep time in a total sleep time.

5. The sleep quality improving agent according to any one of claims 1 to 4, wherein the sleep quality improving agent improves less sleepiness feeling on awakening.

6. The sleep quality improving agent according to any one of claims 1 to 5, wherein the sleep quality improving agent improves ease of falling asleep and feeling of sound sleep.

7. The sleep quality improving agent according to any one of claims 1 to 6, wherein the sleep quality improving agent improves dreaming.

8. The sleep quality improving agent according to any one of claims 1 to 7, wherein the sleep quality improving agent improves feeling of fatigue.

9. The sleep quality improving agent according to any one of claims 1 to 8, wherein the sleep quality improving agent is an oral agent.

10. A sleep quality improving composition comprising the sleep quality improving agent according to any one of claims 1 to 9.

11. The sleep quality improving composition according to claim 10, wherein the sleep quality improving composition is an oral agent.

12. A method of use of an S-adenosylmethionine-containing yeast to improve quality of sleep.

FIG.1

# Fig.2

CUMULATIVE AMOUNT OF ACTIVITY 6 HOURS AFTER ADMINISTRATION

Fig.3

PERCENTAGE OF SLEEP STAGE 4 HOURS AFTER ADMINISTRATION

n=8
**:p<0.01

□ COMPARATIVE EXAMPLE
■ EXAMPLE

PERCENTAGE OF STAGE (%)

WAKEFULNESS        NREM SLEEP

# Fig.4

SLEEP ONSET LATENCY

Fig.5

POWER VALUE OF DELTA WAVES
AT INITIAL STAGE OF SLEEP

# Fig.6

FACTOR I (LESS SLEEPINESS ON AWAKENING)

PERCENT CHANGE (%)

80
60
40
20
0
-20

SAMPLE IN COMPARATIVE EXAMPLE

SAMPLE IN EXAMPLE

Fig.7

FACTOR II (INITIATION AND MAINTENANCE OF SLEEP)

# Fig.8

FACTOR III (STATE OF DREAMING)

PERCENT CHANGE (%)

SAMPLE IN COMPARATIVE EXAMPLE

SAMPLE IN EXAMPLE

Fig.9

FACTOR IV (RECOVERY FROM FATIGUE)

SAMPLE IN COMPARATIVE EXAMPLE

SAMPLE IN EXAMPLE

Fig.10

FEELING OF FATIGUE

## INTERNATIONAL SEARCH REPORT

| | International application No. |
|---|---|
| | PCT/JP2012/076098 |

| A. CLASSIFICATION OF SUBJECT MATTER |
|---|
| *A61K36/06*(2006.01)i, *A61P25/20*(2006.01)i, *A61K31/7076*(2006.01)n |

According to International Patent Classification (IPC) or to both national classification and IPC

| B. FIELDS SEARCHED |
|---|

Minimum documentation searched (classification system followed by classification symbols)
A61K36/06, A61P25/20, A61K31/7076

| Documentation searched other than minimum documentation to the extent that such documents are included in the fields searched |
|---|
| Jitsuyo Shinan Koho          1922-1996    Jitsuyo Shinan Toroku Koho    1996-2012 |
| Kokai Jitsuyo Shinan Koho    1971-2012    Toroku Jitsuyo Shinan Koho    1994-2012 |

Electronic data base consulted during the international search (name of data base and, where practicable, search terms used)
CAPLUS/MEDLINE/EMBASE/BIOSIS(STN), JSTPlus/JMEDPlus/JST7580(JDreamII)

| C. DOCUMENTS CONSIDERED TO BE RELEVANT |
|---|

| Category* | Citation of document, with indication, where appropriate, of the relevant passages | Relevant to claim No. |
|---|---|---|
| X,Y | WO 2009/081833 A1  (Kaneka Corp.),<br>02 July 2009 (02.07.2009),<br>entire text<br>& US 2011/0052623 A1 | 1-11,1-11 |
| X,Y | Stephen Kent et al., Fever Alters<br>Characteristics of Sleep in Rats, Physiology &<br>Behavior, 1988, Vol.44, pp.709-715 | 1-11,1-11 |
| X,Y | JP 2006-62998 A  (Ryukyu Bio-Resource<br>Development Co., Ltd.),<br>09 March 2006 (09.03.2006),<br>entire text; particularly, examples<br>(Family: none) | 1-11,1-11 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>30 October, 2012 (30.10.12) | Date of mailing of the international search report<br>06 November, 2012 (06.11.12) |
|---|---|

| Name and mailing address of the ISA/<br>Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2012/076098 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | Maggini C et al., A polygraph evaluation of the effects of S-adenosyl-L-methionine (SAMe) on all-night sleep, Monographien aus dem Gesamtgebiete der Psychiatrie, 1978, Vol.18, pp.151-69, CAPLUS(AN 1979:615), abstract | 1-11 |
| Y | H. Volkmann et al., Double-blind, Placebo-controlled Cross-over Study of Intravenous S-Adenosyl-L-Methionine in Patients with Fibromyalgia, Scandinavian journal of rheumatology, 1997, Vol.26, pp.206-211 | 1-11 |
| Y | Kazunaga YAZAWA, "SAM-e (S-adenosylmethionine) and locomotive syndrome", Food STYLE 21, 01 October 2011 (01.10.2011), vol.15, no.10, pages 40 to 43 | 1-11 |

Form PCT/ISA/210 (continuation of second sheet) (July 2009)

**INTERNATIONAL SEARCH REPORT**

| International application No. |
| --- |
| PCT/JP2012/076098 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 12
   because they relate to subject matter not required to be searched by this Authority, namely:
   Claim 12 involves "method for treatment of the human body or animal body by surgery or therapy".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006028051 A
- JP 2006062998 A
- JP 2005097101 A
- JP 2007277207 A
- JP 2006513214 PCT

**Non-patent literature cited in the description**

- **YOUNG HO RHEE et al.** *Psychopharmacology,* 1990, vol. 101, 486-488
- *Monogr Gesamtgeb Psychiatr Psychiatry Ser,* 1978, vol. 18, 151-169
- **YAMAMOTO YUKARI ; TANAKA HIDEKI ; TAKASE MIKI ; YAMAZAKI KATSUO ; AZUMI KAZUO ; SHIRAKAWA SHUICHIRO.** Standardization of revised version of OSA sleep inventory for middle age and aged. *Brain science and mental disorders,* 1999, vol. 10, 401-409